# EUROPÄISCHE PATENTSCHRIFT

(11) **EP 1 714 628 B1**
(45) Veröffentlichungstag und Bekanntmachung des Hinweises auf die Patenterteilung: **06.06.2007**
(21) Anmeldenummer: 06007795.5
(22) Anmeldetag: 13.04.2006
(51) Int. Cl.: A61F 17/00

(54) **Erste-Hilfe-Material-Einheit, Verfahren sowie Verbandsmaterialbehältnis**
First-aid kit, method and box for first-aid kit
Trousse de premiers soins, sa fabrication et la boite

(30) Priorität: 18.04.2005 DE 102005017945
(43) Veröffentlichungstag der Anmeldung: 25.10.2006
(73) Patentinhaber: Paul Hartmann AG, 89522 Heidenheim (DE)
(72) Erfinder: Mangold, Rainer, Dr., 89542 Herbrechtingen (DE); Römpp, Angela, 73119 Zell u. A. (DE); Kumpf, Siegfried, Dr., 73340 Amstetten (DE)
(74) Vertreter: Dreiss, Fuhlendorf, Steimle & Becker

(56) Entgegenhaltungen:
- EP-A- 1 174 105
- EP-A- 1 388 325
- DE-A1- 10 105 115
- DE-A1- 10 125 423
- DE-U1- 9 313 406
- GB-A- 792 087

## Beschreibung

Die Erfindung betrifft eine Erste-Hilfe-Material-Einheit insbesondere zur Verwendung in Verbandsmaterialbehältnissen in Kraftfahrzeugen umfassend eine die Erste-Hilfe-Material-Einheit allseitig umschließende Hülle sowie in die Hülle aufgenommenes Erste-Hilfe-Material.

Mit Erste-Hilfe-Material-Einheiten sind Zusammenstellungen mehrerer Erste-Hilfe-Materialien, wie Verbandspäckchen, Kompressen, Pflasterrollen, etc. gemeint, die gemeinsam konfektioniert sind.

Derartige Exste-Hilfe-Materxal-Einheiten, aber auch Verbandsmaterialbehältnisse sind im Stand der Technik vielfach bekannt und werden beispielsweise in der DIN 13164 sowie in der DIN 13167 definiert bezüglich ihrer in derartigen Materialeinlzeiten enthaltenen Gegenstände und Utensilien sowie weiterer Parameter. Auf der anderen Seite besteht der Wunsch und auch die Notwendigkeit seitens der Herszeiler von Kraftfahrzeugen und Krafträdern, Erste-Hilfe-Materialien möglichst volumenreduziert bereitzustellen, da der Wunsch von Autokäufern in Richtung eines immer größeren Innenraumangebotes geht, wodurch jedoch Stauräume für Verbandsmaterialien, aber auch Warndreiecke etc. hinsichtlich des Platzangebotes reduziert werden. Aufgrund der durch die entsprechende deutsche Industrienorm vorgeschriebenen in einem Erste-Hilfe-Material-Verbandskasten enthaltenen Gegenstände lässt sich jedoch eine Volumenreduktion nicht durch Änderung der Zusammenstellung der Erste-Hilfe-Materialien erzielen.
Darüber hinaus macht die entsprechende Norm auch Angaben darüber, wie die einzelnen Bestandteile eines Erste-Hilfe-Material-Verbandskasten verpackt sein müssen, insbesondere steril oder staubgeschützt.

Im Stand der Technik sind nun in der Vergangenheit vielfach Bemühungen unternommen worden, das Erste-Hilfe-Material insbesondere in Kraftfahrzeugen bedarfsgerecht zu verpacken und bereitzustellen. So offenbart beispielsweise die DE 101 05 115 A1 einen Verbandsmaterialbehälter zur Verwendung in Kraftfahrzeugen mit einem Behälterunterteil zur Aufnahme von Erste-Hilfe-Material sowie einem Behälterdeckel, in dem ebenfalls Erste-Hilfe-Material angeordnet ist, das mit dem Deckel in eine geöffnete Position des Behälters verschwenkt werden kann, so dass das Erste-Hilfe-Material besonders übersichtlich für einen Benutzer dargeboten ist. Darüber hinaus kann beispielsweise gemäß der Druckschrift vorgesehen sein, dass das Erste-Hilfe-Material in ein steriles und in ein nicht steriles Modul unterteilt ist und jeweils eins der Module einer Behälterhälfte zugeordnet ist.

Des Weiteren ist beispielsweise aus der DE 101 25 423 A1 eine Erste-Hilfe-Einrichtung für Kraftfahrzeuge bekannt, mit einer elastischen und/oder aufrollbaren Matte, die gleichzeitig den Träger für die Gegenstände der Erste-Hilfe-Ausrüstung bietet. Dabei soll die Matte so zusammenklappbar sein, dass ein zylinderförmiger Behälter ausgebildet wird. Auf diese Weise soll eine Erste-Hilfe-Einrichtung geschaffen werden, die kompakt gebaut und andererseits einfach in der Handhabung ist.

In den letzten Jahren ist nun zunehmend ein Trend zu immer kleiner werdenden Erste-Hilfe-Behältnissen festzustellen. Die Erfindung stellt sich daher die Aufgabe, in diesem Spannungsfeld aus geringem Platzangebot beziehungsweise Vorgaben der Automobilhersteller bezüglich des Raumes für eine Erste-Hilfe-Material-Einheit und auf der anderen Seite den Voraussetzungen hinsichtlich der in einer solchen Einheit befindlichen Materialien, eine Erste-Hilfe-Material-Einheit bereitzustellen, die eine weitere Volumenreduktion ermöglicht.

Die Erfindung löst dabei diese Aufgabe unter anderem durch Bereitstellung einer Erste-Hilfe-Material-Einheit, bei der die Hülle aus einem gasdichten Material besteht und das Innere zur Volumenreduktion evakuiert ist.
Dabei hat sich gezeigt, dass gegenüber den bisher verwendeten Tiefziehpackungen, die zweiteilig gestaltet sind und aus einer unteren im Tiefziehverfahren hergestellten Wanne zur Aufnahme der Erste-Hilfe-Materialien bestehen sowie einem damit allseitig verschweißten Deckel, das Volumen deutlich reduziert werden kann.

Dabei ist insbesondere eine Volumenreduktion auf ein Volumen, das dem theoretisch errechneten Volumen der in der Einheit verpackten Gegenstände entspricht, wünschenswert. Besonders vorzugsweise kann das Volumen jedoch noch unter dieses Volumen reduziert werden, insbesondere um > 5%, vorzugsweise um >10 % unter diesen wert.

Die Hülle ist dabei wünschenswerterweise nicht nur gassondern auch flüssigkeitsdicht.

Maschinen zur Verpackung von derartigen Einheiten und insbesondere zur Evakuierung entsprechender Einheiten sind beispielsweise aus der WO 2004/000650 A1, aber auch aus der EP 710 605 B1 bekannt.

Dabei kann es zur weiteren Volumenreduktion, die sogar das theoretisch errechnete Volumen der Einzelbestandteile unterschreiten kann, vorgesehen sein, dass die Einzelverpackungen der in die Hülle aufgenommenen Erste-Hilfe-Materialien gasdurchlässig ausgebildet sind. Die Materialien, insbesondere die steril verpackten Materialien, sind standardmäßig in gasdurchlässigen Verpackungen vorgesehen. Es kann nun weiter vorgesehen sein, auch die unsterilen Bestandteile gemäß den vorgesehenen DIN-Normen in gasdurchlässige Verpackungen, insbesondere Folienmaterialien, die nach wie vor jedoch staubdicht ausgebildet sind, zu verpacken, so dass dann diese Verpackungen bei der Evakuierung der Hülle ebenfalls mit evakuiert werden, so dass das Gesamtvolumen der Erste-Hilfe-Material-Einheit dann die Summe der theoretischen Volumina der Einzelkomponenten sogar noch unterschreiten kann. Schließlich kann es weiterhin vorgesehen sein, dass eine weitere Volumenreduktion durch Verpressen während oder vor dem Evakuiervorgang erfolgen kann. Auf diese weise kann der Evakuiervorgang durch von außen aufgebrachten Druck verbessert und beschleunigt werden. Zwischenräume werden so weiter reduziert, ohne dass durch übermäßiges Vakuum die Einheit stark verhärtet wird.

Bevorzugt ist es vorgesehen, dass das Innere der Hülle auf einen Druck < 600 mbar, insbesondere < 500 mbar und insbesondere < 400 mbar und insbesondere vorzugsweise auf einen Innendruck von 350 - 250 mbar und besonders bevorzugt auf einen Innendruck von 300 mbar evakuiert wird. Dabei hat sich gezeigt, dass bei einer Evakuierung auf einen Innendruck > 600 mbar keine nennenswerte Volumenreduktion erfolgt. Durch eine Evakuierung auf ca. 600 mbar kann eine. Volumenreduktion um ca. 10 % und bei 500 mbar von ungefähr 24 % erreicht werden. Eine derartige Volumenreduktion von ca. 24 % entspricht einer Volumenreduktion, wie sie ebenfalls dadurch erreicht werden kann, dass die einzelnen Artikel und Gegenstände der Erste-Hilfe-Material-Einheit manuell besonders.platzsparend und optimiert angeordnet werden, was aus dem Stand der Technik bekannt ist. Durch eine Druckabsenkung auf ca. 400 mbar in den Innenhülle kann eine deutliche Volumenreduktion um ca. 35 % erreicht werden. Besonders bevorzugt ist eine Volumenreduktion auf einen Innendruck von 300 mbar, da hierbei eine deutliche Volumenreduktion erreicht wird, Inhaltsteile jedoch nicht beschädigt werden. Bei einer weiteren Reduktion und Evakuierung auf < 300 mbar kann es zu einer Verformung beispielsweise der in der Einheit vorgesehenen Pflasterspule, aber auch zur deutlichen Verformung einzelner anderer Inhaltsteile kommen. Wird der Innendruck dann noch weiter abgesenkt, kommt es zum Aufreißen einzelner Verpackungen und einzelne Inhaltsstoffe werden stark geknickt. Insbesondere kann es zur Verletzung der Sterilität einzelner Verpackungen kommen. Darüber hinaus bietet eine Volumenreduktion in der bevorzugten Größe den Vorteil, dass die verbandsmaterialeinheit Hülle noch eine gewisse Flexibilität aufweist und noch nicht zu einer völlig starren brettartigen Einheit verhärtet ist. Auf diese weise lässt sich die Einheit später besser in ein Verbandsmaterialbehältnis einfügen und insbesondere lässt sich ein Verbandsmaterialbehältnis, in das die Einheit aufgenommen worden ist, auch leichter in zum Teil enge oder unregelmäßig geformte Hohlräume eines Kraftfahrzeuges einbringen.

Es kann dabei vorgesehen sein, dass die Hülle insbesondere aus einer Folie, insbesondere aus Polyamid (PA), polyvinylalkohol (PVAL), Polyester (PES), polyethylenterephthalat (PET) oder Polyethylen (PE) besteht, wobei insbesondere auch Verbundfolien, so insbesondere zum Beispiel aus PA/PVAL/PE vorgesehen sein können. Die Dicke der Folien kann dabei zwischen 150 und 200 µm liegen, insbesondere 160 - 180 µm und vorzugsweise bei 170 µm. Es kann weiterhin vorgesehen sein, um eine Sauerstoffundurchlässigkeit der Folie und damit der Hülle sicherzustellen, dass das Folienmaterial mit einer Aluminiumbeschichtung (A1) versehen ist, wobei die Aluminiumbeschichtung eine Auftragsdicke im Bereich von 5 - 15 µm besitzt. Dabei ist insbesondere vorgesehen ein Verbundmaterial aus PE/Al/PET mit einer Schichtdicke von 70/7/12 µm einzusetzen.

Sofern eine Aluminiumbeschichtung vorgesehen ist, ist die Verpackungsfolie, also die Hülle, nicht transparent. Ansonsten kann es vorgesehen sein, transparente Folien einzusetzen, damit einem Verwender einer Erste-Hilfe-Material-Einheit in einem Kraftfahrzeug die einzelnen Materialelemente, aber auch die Einheit als solche besser ins Auge fällt, da er die Beschriftung auf den einzelnen Materialien durch die Hülle hindurch erkennen kann. Darüber hinaus muss das Material und insbesondere die Folie für die Hülle neben einer Gasdichtheit insbesondere auch eine ausreichende Bruchfestigkeit insbesondere auch bei verformung der Erste-Hilfe-Material-Einheit aufweisen, um das Vakuum im Inneren der Hülle über lange Zeit sicherzustellen und die darin aufgenommenen Erste-Hilfe-Materialien gegen mechanische, aber auch chemische und thermische Einwirkung von außen zu schützen.

Es kann dabei vorgesehen sein, dass sowohl sterile als auch unsterile Materialien des Erste-Hilfe-Materials in einer gemeinsamen Einheit aufgenommen sind.

Als Hülle kann beispielsweise vorgesehen sein, eine Schlauchfolie vorzusehen, die an mindestens einer Seite zum Befüllen eine Öffnung aufweist. Grundsätzlich sind jedoch auch andere Gestaltungen beispielsweise aus zwei Folienbahnen denkbar, die vor oder nach dem Befüllen miteinander verbunden werden und so eine Tasche bilden, die befüllt werden kann. Die Hülle wird in ihrem Öffnungsbereich in aller Regel über eine Siegelnaht verschlossen werden, indem die beiden Kunststofffolien miteinander thermisch oder durch sonst bekannte Verfahren, zum Beispiel Ultraschallschweißen verschweißt werden.

Besonders vorteilhaft kann vorgesehen sein, dass die Hülle aus einem thermisch schrumpfbaren Material besteht. Durch die Schrumpfung des Hüllmaterials kann erreicht werden, dass keine durch die Siegelnähte gebildeten überstehenden Ränder vorhanden sind, wodurch die Einheit dann besser in ein Verbandsmaterialbehältnis eingefügt werden kann. Darüber hinaus können die relativ harten Siegelränder einer Verpackung gegebenenfalls deren Handhabung beeinträchtigen. Die Schrumpfung kann dabei unmittelbar nach dem Verschließen erfolgen, wobei die Schrumpfung über eine thermische Beaufschlagung der Einheit erfolgt.

Des Weiteren kann erfindungsgemäß vorgesehen sein, dass die Hülle eine Aufreiß- beziehungsweise Öffnungshilfe aufweist, wobei eine solche insbesondere in einer Kerbe in der die Hülle verschließenden Siegelnaht bestehen kann. Alternativ können auch Materialschwächungen oder sonstige Sollbruchstellen vorgesehen sein, die ein Öffnen erleichtern, indem die Öffnungskraft herabgesetzt wird. Es können auch vorteilhaft Anfassbereiche vorgesehen sein, die das Ergreifen und damit das Öffnen erleichtern.

Die Erfindung betrifft des Weiteren ein Verfahren zum volumenreduzierten Verpacken von Erste-Hilfe-Material, insbesondere zur Verwendung in einem Verbandsmaterialbehältnis eines Kraftfahrzeugs mit den Schritten Bereitstellen einer mindestens bereichsweise offenen Hülle, Befüllen der Hülle mit den Erste-Hilfe-Materialien und Evakuieren des Hülleninneren auf einen definierten Hülleninnendruck und Verschließen der Hüllenöffnung, so dass die Hülle evakuiert verbleibt.

Es kann dabei vorgesehen sein, dass gleichzeitig mit oder vor dem Evakuieren ein Verpressen des in der Hülle befindlichen Erste-Hilfe-Materials erfolgt. In einer weiteren Ausgestaltung des Verfahrens kann das Hüllenmaterial zusätzlich einem Schrumpfungsprozess unterzogen werden. Gemäß einem besonders bevorzugten Verfahren ist vorgesehen, dass gleichzeitig mit oder vor dem Evakuieren ein Verpressen des in der Hülle befindlichen Erste-Hilfe-Materials erfolgt und zusätzlich das Hüllenmaterial nach dem Verschließen der Hüllenöffnung einem Schrumpfungsprozess unterzogen wird.

Schließlich betrifft die Erfindung noch ein Verbandsmaterialbehältnis zur Aufnahme einer vorstehend beschriebenen Erste-Hilfe-Material-Einheit. Hierbei kann vorgesehen sein, dass die Hülle selbst das Verbandsmaterialbehältnis bildet. Weitere Vorteile und Merkmale der Erfindung ergeben sich aus den übrigen Anmeldungsunterlagen. Die Erfindung soll im Folgenden anhand von Beispielen näher erläutert werden.

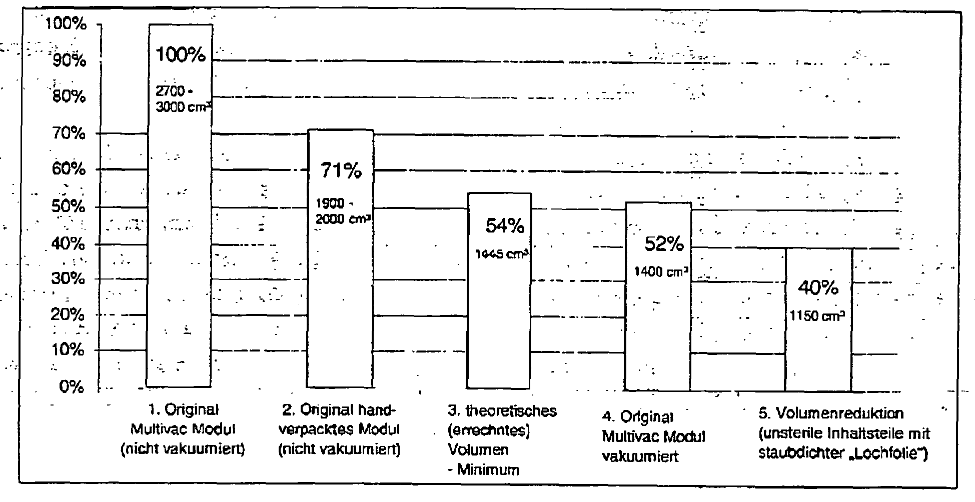

So zeigt vorstehende Abbildung ein Diagramm, bei dem eine herkömmliche von der Paul Hartmann AG vertriebene, nicht vakuumierte Erste-Hilfe-Material-Einheit, hier als Multivac Modul bezeichnet, die ein Volumen zwischen 2.700 und 3.000 cm³ aufweist als Bezugsgröße zu 100 % gesetzt worden ist.

Hierbei handelt es sich um eine maschinelle Modulpackung, wobei die Hülle im Tiefziehverfahren mit den Maßen L x B x H [mm] 240 x 150 x 70 mm verwendet wurde. Die Inhaltsteile werden in zwei Tiefziehpackungen getrennt nach steril und unsteril eingelegt und mit einer Folie als Oberbahn durch Verschweißung verschlossen. Dem Diagramm lässt sich nun in der zweiten Säule entnehmen, dass durch bereits entsprechend sorgfältige Verpackung, wobei anstelle der maschinellen Modulpackung eine manuelle Modulpackung verwendet wird und die Inhaltsteile ebenso wie bei der ersten Säule getrennt nach steril und unsteril eingelegt werden und mittels Adhäsionsverschluss verschlossen werden, eine Volumenreduktion auf ca. 71 % und ein absolutes Volumen von 1.900 - 2.000 cm³ möglich wird. Eine derartige manuelle Packung der Module stellt eine übliche Vorgehensweise zur Volumenreduktion dar, bedingt jedoch einen erheblichen personellen Aufwand.

Die dritte Säule stellt das theoretisch.errechnete Volumenminimum dar, für dessen Bestimmung die Volumina aller Inhaltsteile addiert worden sind. Dabei sind die unsterilen Inhaltsteile gasdicht verpackt. Die Inhaltsteile der Erste-Hilfe-Material-Einheit machen dabei ca. 54 % des Gesamtinhaltes aus.

Derartige Volumenmessungen erfolgen dabei mittels eines Verdrängungstests von Wasser nach der Überlaufmethode, da die Hüllen wasserundurchlässig sind. Die Volumina werden durch Untertauchen der Probekörper in ein mit Wasser gefülltes Überlaufgefäß gemessen. Dieses Gefäß kann nur eine begrenzte Wassermenge aufnehmen, da überschüssiges Wasser durch ein seitlich angebrachtes Röhrchen ablaufen kann. Der Wasserspiegel wird vor der Messung bis zur Unterkante des Überlaufröhrchens eingestellt, so dass beim Eintauchen eines Probenkörpers soviel Wasser über das Überlaufröhrchen abfließt, wie dar Probenkörper Volumen besitzt. Die übergelaufene Flüssigkeitsmenge wird aufgefangen und gravimetrisch bestimmt (Dichte Wasser (20 °C = 0,99820 g/ ml). Zum Untertauchen wird ein stumpfer Glasstab verwendet. Das Volumen des eingetauchten Glasstabes wird bei der Volumenbestimmung der Probekörper berücksichtigt.

Die vierte Säule zeigt nun eine erstmals vakuumierte Erste-Hilfe-Material-Einheit, bei der die äußere Hülle durch eine Verbundfolie aus PA/PVAL/PE gebildet worden ist und die Folie eine Stärke von 170 µm aufweist. Die Einheit wurde dann beim Verschließen evakuiert. Man erhält hier eine Volumenreduktion um. 48 % gegenüber der Ausgangseinheit.
Dass die Volumenreduktion sogar noch unter dem theoretisch errechneten Volumenminimum liegt, liegt daran, dass die Verpackungen der sterilen Inhaltsteile gasdurchlässig sind, so dass auch diese Verpackungen der Inhaltsteile mit evakuiert werden können.

Um nun noch eine weitere Volumenreduktion zu erreichen, ist erfindungsgemäß weiterhin vorgesehen worden, auch die bisher luftdicht verpackten unsterilen Inhaltsteile mit einer staubdichten, luftdurchlässigen Folie auszustatten.
Beim Evakuieren werden dann auch diese Teile im Volumen reduziert, so dass eine insgesamte Reduktion um 60 % des Ausgangsvolumens erreicht werden kann.

Eine weitere Volumenreduktion kann noch durch gleichzeitiges Verpressen der Inhaltsteile erfolgen.
Besonders vorteilhaft kann vorgesehen sein, dass die Folie für die Hülle eine Schrumpffolie ist und diese unmittelbar nach dem Verschließen der Hülle geschrumpft wird, da sich.. hierdurch eine besonders faltenfreie und insbesondere eine Umhüllung der Erste-Hilfe-Material-Einheit ohne störende deutlich abstehende Verschlussränder der Hülle ergibt.

Die erfolgte Volumenreduktion in den Erste-Hilfe-Material-Einheiten kann dann, wenn auch nicht vollständig, an ein Verbandsmaterialbehältnis weitergegeben werden, das die Einheit aufnimmt, das dadurch ebenfalls geringere Abmessungen aufweisen kann.

Dies soll anhand einer nachfolgenden Tabelle noch einmal verdeutlich werden, die in der ersten Spalte den Innendruck der verwendeten Erste-Hilfe-Material-Einheit angibt, die hier mit "Modul" bezeichnet ist. Die zweite Spalte zeigt nun das Volumen des vakuumierten Moduls in cm³ an, wobei in der dritten Spalte zum einen die Volumenreduktion in cm³ und zum anderen in Prozent angegeben ist. In der vierten Spalte sind Bemerkungen angegeben, wobei erkannt werden kann, dass eine Evakuierung auf weniger als 300 mbar zu einer Verformung der Inhaltsteile der Erste-Hilfe-Material-Einheit führen kann,die insbesondere im Fall des Aufplanzens von Innenverpackungen nicht erwünscht ist. Dabei kommen als Innenverpackungen generell solche in rage und sind besonders vorteilhaft, die ebenfalls gasdurchlässig sind und auf diese Weise mit evakuiert werden können. Alternativ können bereits vakuumverpackte Inhaltsstoffe eingesetzt werden, wie dies bereits bei sogenannten Verbandsmaterialpäckchen üblich ist.

| [mbar] | Volumen Modul vakuumiert [cm³] | Volumenreduktion Multivac Vakuum/ Multivac Original | | Bemerkungen |
|---|---|---|---|---|
| | | [cm³] | [%] | |
| 698 | 2690 | - | - | wie Multivac original |
| 600 | 2380 | 310 | 11,5 | |
| 500 | 2040 | 650 | 24,2 | wie handgepacktes Modul |
| 400 | 1740 | 950 | 35,3 | |
| 300 | 1400 | 1290 | 47,9 | deutliche Volumenreduktion, Inhalt unbeschädigt |
| 300 Schrumpfung | 1400 | 1290 | 47,9 | keine überstehenden Ränder, einfacher in Verbandtasche einzulegen |
| 250 | 1170 | 1520 | 56,5 | Pflasterspule leicht verformt |
| 200 | 1040 | 1650 | 61,3 | Module und einzelne Inhaltsteile leicht verformt |
| 200 Schrumpfung | 1020 | 1670 | 62,1 | keine überstehenden Ränder |
| 150 | 890 | 1870 | 66,9 | Pflasterspule stark verformt, Rettungsdecke verformt und viele Knicke |
| 100 | 820 | 1870 | 69,5 | Verpackung geplatzt, Binden, Rettungsdecke, Verbandspäckchen, Kompressen haben sehr starke Knicke |
| 50 | 740 | 1950 | 72,5 | Module verformen sich in Schalenform und sind sehr hart, Verpackung geplatzt, Sterilbruch |
| 5 | 685 | 2005 | 74,5 | Module verformen sich in Schalenform und sind sehr hart, Verpackung geiplätzt, Sterllbruch, Pflasterspule stark verformt, Pflasterschichten lösen sich ab |

Aus der vorstehenden Tabelle ergibt sich daher, dass eine Evakuierung auf einen Innendruck von 300 mbar besonders vorteilhaft ist, da sich eine größtmögliche Volumenreduktion bei gleichzeitiger Nichtbeschädigung der Inhaltsteile ergibt. Sofern gewünscht ist, dass keine überstehenden Ränder entstehen und auf diese Weise die Einheit leichter in eine Tasche eingelegt werden kann, kann eine Schrumpfung vorgesehen sein. Bei der Schrumpfung erfolgt keine weitere oder nur zu vernachlässigende Volumenreduktion.

Insgesamt kann auf vorstehend beschriebene weise, wobei die Evakuierung und das Verpressen gemäß im Stand der Technik beschriebenen Verfahren erfolgt, ein deutlich volumenreduzierte Erste-Hilfe-Material-Einheit bereitgestellt werden, die trotzdem eine ausreichende Flexibilität besitzt, um in enge beziehungsweise unregelmäßig geformte Stauräume eines Kraftfahrzeugs eingelegt werden zu können.

## Patentansprüche

1. Erste-Hilfe-Material-Einheit insbesondere zur Verwendung in Verbandsmaterialbehältnissen in Kraftfahrzeugen umfassend eine die Erste-Hilfe-Material-Einheit allseitig umschließende Hülle sowie in die Hülle aufgenommenes Erste-Hilfe-Material, **dadurch gekennzeichnet, dass** die Hülle aus einem gasdichten Material besteht und das Innere der Hülle zur Volumenreduktion evakuiert ist.

2. Erste-Hilfe-Material-Einheit nach Anspruch 1, **dadurch gekennzeichnet, dass** das in die Hülle aufgenommene Material einzelverpackt ist und die Einzelverpackungen gasdurchlässig ausgebildet sind.

3. Erste-Hilfe-Material-Einheit nach Anspruch 1 oder 2, **dadurch gekennzeichnet, dass** die Einheit zur Reduzierung des Volumens verpresst ist.

4. Erste-Hilfe-Material-Einheit nach einem der vorangehenden Ansprüche, **dadurch gekennzeichnet, dass** das Innere der Hülle auf einen Druck kleiner 600 mbar, insbesondere kleiner 500 mbar, insbesondere kleiner 400 mbar und insbesondere auf einen Innendruck von 350-250 mbar und insbesondere 300 mbar evakuiert ist.

5. Erste-Hilfe-Material-Einheit nach einem der vorangehenden Ansprüche, **dadurch gekennzeichnet, dass** die Hülle aus einer Folie, insbesondere aus PA, EVOH, PES, PET oder PE besteht.

6. Erste-Hilfe-Material-Einheit nach Anspruch 5, **dadurch gekennzeichnet, dass** die Folie eine Verbundfolie ist.

7. Erste-Hilfe-Material-Einheit nach Anspruch 5 oder 6, **dadurch gekennzeichnet, dass** die Folie eine Dicke von 150 bis 200 µm, insbesondere von 170 µm aufweist.

8. Erste-Hilfe-Material-Einheit nach einem der Ansprüche 5-7, **dadurch gekennzeichnet, dass** die Folie aluminiumbeschichtet ist und insbesondere die Aluminiumbeschichtung eine Dicke von 5 bis 15 µm aufweist.

9. Erste-Hilfe-Material-Einheit nach einem der vorangehenden Ansprüche, **dadurch gekennzeichnet, dass** das aufgenommene Erste-Hilfe-Material steriles und unsteriles Material enthält.

10. Erste-Hilfe-Material-Einheit nach einem der vorangehenden Ansprüche, **dadurch gekennzeichnet, dass** die Hülle wenigstens bereichsweise eine Siegelnaht beinhaltet.

11. Erste-Hilfe-Material-Einheit nach einem der vorangehenden Ansprüche, **dadurch gekennzeichnet, dass** die Hülle aus einem thermisch schrumpfbaren Material besteht.

12. Erste-Hilfe-Material-Einheit nach einem der vorangehenden Ansprüche, **dadurch gekennzeichnet, dass** die Hülle eine Öffnungshilfe aufweist.

13. verfahren zum volumenreduzierten Verpacken von Erste-Hilfe-Material in einer Erste-Hilfe-Material-Einheit nach einem der vorstehenden Ansprüche insbesondere zur Verwendung in einem Verbandsmaterialbehältnis eines Kraftfahrzeugs mit den Schritten, Bereitstellen einer mindestens bereichsweise offenen Hülle, Befüllen der Hülle mit den Erste-Hilfe-Materialien, Evakuieren des Hülleninneren auf einen definierten Hülleninnendruck und Verschließen der Hüllenöffnung.

14. Verfahren nach Anspruch 13, **dadurch gekennzeichnet, dass** gleichzeitig mit oder vor dem Evakuieren ein Verpressen des in der Hülle befindlichen Erste-Hilfe-Materials erfolgt.

15. Verbandsmaterialbehältnis umfassend eine Erste-Hilfe-Materialeinheit nach einem der Ansprüche 1 bis 12.

## Claims

1. First-aid material unit, in particular for use in dressing material containers in motor vehicles comprising a casing enclosing the first-aid material unit on all sides and also first-aid material incorporated into the casing, **characterised in that** the casing consists of a gas-tight material and the interior of the casing is evacuated to reduce the volume.

2. First-aid material unit according to claim 1, **characterised in that** the material incorporated into the casing is individually packaged and the individual packages are configured to be gas-permeable.

3. First-aid material unit according to either claim 1 or claim 2, **characterised in that** the unit is compressed to reduce the volume.

4. First-aid material unit according to any one of the preceding claims, **characterised in that** the interior of the casing is evacuated to a pressure of less than 600 mbar, in particular less than 500 mbar, especially less than 400 mbar and in particular to an internal pressure of from 250 to 350 mbar and especially 300 mbar.

5. First-aid material unit according to any one of the preceding claims, **characterised in that** the casing consists of a film, in particular of PA, EVOH, PES, PET or PE.

6. First-aid material unit according to claim 5, **characterised in that** the film is a composite film.

7. First-aid material unit according to either claim 5 or claim 6, **characterised in that** the film has a thickness of from 150 to 200 µm, in particular of 170 µm.

8. First-aid material unit according to any one of claims 5 to 7, **characterised in that** the film is aluminium-coated and, in particular, the aluminium coating has a thickness of from 5 to 15 µm.

9. First-aid material unit according to any one of the preceding claims, **characterised in that** the incorporated first-aid material contains sterile and unsterile material.

10. First-aid material unit according to any one of the preceding claims, **characterised in that** the casing contains a sealed seam, at least in certain regions.

11. First-aid material unit according to any one of the preceding claims, **characterised in that** the casing consists of a heat-shrinkable material.

12. First-aid material unit according to any one of the preceding claims, **characterised in that** the casing has an opening aid.

13. Method for the volume-reduced packaging of first-aid material in a first-aid material unit according to any one of the preceding claims, in particular for use in a dressing material container of a motor vehicle, including the following steps: providing a casing open at least in certain regions, filling the casing with the first-aid materials, evacuating the interior of the casing to a defined casing internal pressure and sealing the casing opening.

14. Method according to claim 13, **characterised in that** the first-aid material located in the casing is compressed simultaneously with or prior to the evacuation.

15. Dressing material container comprising a first-aid material unit according to any one of claims 1 to 12.

## Revendications

1. Trousse de premiers soins, destinée en particulier à une utilisation dans des boîtes de matériel de bandage à bord de véhicules automobiles, comprenant un étui entourant de tous les côtés la trousse de premiers soins, ainsi que du matériel de premiers soins logé dans l'étui, **caractérisée en ce que** l'étui est constitué d'un matériau étanche au gaz et **en ce que** l'intérieur de l'étui est vidé de son air afin de réduire son volume.

2. Trousse de premiers soins selon la revendication 1, **caractérisée en ce que** le matériel logé dans l'étui est emballé individuellement et **en ce que** les emballages individuels sont conçus perméables au gaz.

3. Trousse de premiers soins selon la revendication 1 ou 2, **caractérisée en ce que** la trousse est comprimée afin de réduire son volume.

4. Trousse de premiers soins selon l'une des revendications précédentes, **caractérisée en ce que** l'intérieur de l'étui est vidé de son air à une pression inférieure à 600 mbar, en particulier inférieure à 500 mbar, en particulier inférieure à 400 mbar et en particulier à une pression interne comprise entre 350 et 250 mbar et en particulier 300 mbar.

5. Trousse de premiers soins selon l'une des revendications précédentes, **caractérisée en ce que** l'étui est constitué d'une feuille de plastique, en particulier en PA, EVOH, PES, PET ou PE.

6. Trousse de premiers soins selon la revendication 5, **caractérisée en ce que** la feuille de plastique est une feuille composite.

7. Trousse de premiers soins selon la revendication 5 ou 6, **caractérisée en ce que** la feuille de plastique présente une épaisseur de 150 à 200 µm, en particulier de 170 µm.

8. Trousse de premiers soins selon l'une des revendications 5 à 7, **caractérisée en ce que** la feuille de plastique est recouverte d'aluminium et en particulier **en ce que** le revêtement en aluminium présente une épaisseur de 5 à 15 µm.

9. Trousse de premiers soins selon l'une des revendications précédentes, **caractérisée en ce que** le matériel de premiers soins logé contient du matériel stérile et non stérile.

10. Trousse de premiers soins selon l'une des revendications précédentes, **caractérisée en ce que** l'étui comprend au moins partiellement un joint soudé.

11. Trousse de premiers soins selon l'une des revendications précédentes, **caractérisée en ce que** l'étui se compose d'un matériau thermiquement rétractable.

12. Trousse de premiers soins selon l'une des revendications précédentes, **caractérisée en ce que** l'étui présente un dispositif d'aide à l'ouverture.

13. Procédé de conditionnement à volume réduit de matériel de premiers soins dans une trousse de premiers soins selon l'une des revendications précédentes, en particulier pour une utilisation dans une boîte de matériel de bandage à bord d'un véhicule automobile, consistant à préparer un étui au moins partiellement ouvert, à remplir cet étui avec le matériel de premiers soins, à vider l'air de l'intérieur de l'étui à une pression interne de l'étui définie et à fermer l'ouverture de l'étui.

14. Procédé selon la revendication 13, **caractérisé en ce qu'**il se produit une compression du matériel de premiers soins se trouvant dans l'étui en même temps ou avant le vide d'air.

15. Boîte de matériel de bandage comprenant une trousse de premiers soins selon l'une des revendications 1 à 12.
